(19) Europäisches Patentamt
European Patent Office
Office européen des brevets

(11) Veröffentlichungsnummer: **0 118 070**
**B1**

# (12) EUROPÄISCHE PATENTSCHRIFT

(45) Veröffentlichungstag der Patentschrift: **08.10.86**

(21) Anmeldenummer: **84101713.0**

(22) Anmeldetag: **20.02.84**

(51) Int. Cl.⁴: **C 07 D 249/08**, C 07 D 233/60, C 07 D 403/06, A 61 K 31/41, A 61 K 31/415 // C07D405/06

## (54) Substituierte 1,3-Diazolyl-2-propanole, Verfahren zu ihrer Herstellung und ihre Verwendung als antimykotische Mittel.

(30) Priorität: **02.03.83 DE 3307217**

(43) Veröffentlichungstag der Anmeldung: **12.09.84 Patentblatt 84/37**

(45) Bekanntmachung des Hinweises auf die Patenterteilung: **08.10.86 Patentblatt 86/41**

(84) Benannte Vertragsstaaten: **AT BE CH DE FR GB IT LI LU NL SE**

(56) Entgegenhaltungen:
**EP - A - 0 044 605**
**EP - A - 0 061 051**
**EP - A - 0 069 442**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Holmwood, Graham, Dr., Krutscheider Weg 105, D-5600 Wuppertal 11 (DE)**
Erfinder: **Regel, Erik, Dipl.-Ing., Untere Bergerheide 26, D-5600 Wuppertal 1 (DE)**
Erfinder: **Büchel, Karl Heinz, Prof. Dr., Dabringhausener Strasse 42, D-5093 Burscheid (DE)**
Erfinder: **Schaller, Klaus, Dr., Am Sonnenschein 38, D-5600 Wuppertal 1 (DE)**
Erfinder: **Plempel, Manfred, Dr., Zwengenbergstrasse 3c, D-5657 Haan (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte 1,3-Diazolyl-2-propanole, ein Verfahren zu ihrer Herstellung und ihre Verwendung bei der Behandlung von Krankheiten, insbesondere von Mykosen.

Aus EP-A 44 605 ist bereits bekannt geworden, dass bestimmte 1,3-Diazolyl-2-propanole fungizide Eigenschaften aufweisen. Bei der antimykotischen Anwendung erwiesen sie sich jedoch als nicht befriedigend.

Es wurden neue substituierte 1,3-Diazolyl-propanole der allgemeinen Formel

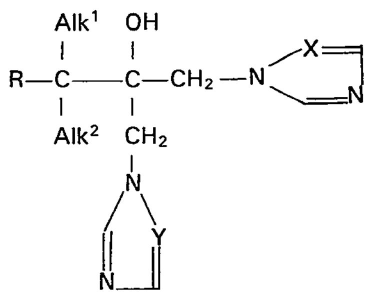

(I)

in welcher

Alk[1] für geradkettiges oder verzweigtes Alkyl mit 1 bis 4 Kohlenstoffatomen;

Alk[2] für geradkettiges oder verzweigtes Alkyl mit 1 bis 4 Kohlenstoffatomen;

Alk[1] und Alk[2] gemeinsam für einen 3- bis 7gliedrigen cycloaliphatischen Ring;

X für ein Stickstoffatom oder die CH-Gruppe;

Y für ein Stickstoffatom oder die CH-Gruppe; und

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroxyiminoalkyl, Alkoxyiminoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy stehen,

und deren physiologisch verträgliche Säureadditionssalze gefunden.

Bevorzugt sind diejenigen Verbindungen der Formel (I), in denen

Alk[1] für Methyl oder Ethyl steht;

Alk[2] für Methyl oder Ethyl steht;

Alk[1] und Alk[2] gemeinsam mit dem Kohlenstoffatom, an das sie gebunden sind, für Cyclobutyl, Cyclopentyl oder Cyclohexyl stehen;

X für ein Stickstoffatom oder die CH-Gruppe steht;

Y für ein Stickstoffatom oder die CH-Gruppe steht; und

R für jeweils gegenebenfalls im Phenylteil einfach oder zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten genannt seien: Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroxyiminomethyl, 1-Hydroxyiminoethyl, sowie jeweils gegebenenfalls durch Fluor, Chlor, Methyl, substituiertes Phenyl, Phenoxy, Benzyl und Benzyloxy.

Bevorzugte erfindungsgemässe Verbindungen sind auch Additionsprodukte aus Säuren und denjenigen substituierten 1,3-Diazolyl-2-propanolen der Formel (I), in denen die Substituenten Alk[1], Alk[2], X, Y und R die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolksulfonsäure mit 1,5-Naphthalindisulfonsäure.

Weiterhin wurde gefunden, dass man die substituierten 1,3-Diazolyl-2-propanole der Formel (I) erhält, wenn man 2-Azolylmethyl-oxirane der Formel

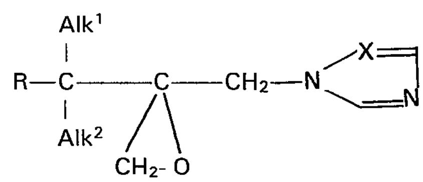

(II)

in welcher

Alk[1], Alk[2], R und X die oben angegebene Bedeutung haben, mit Azolen der Formel

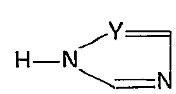

(III)

in welcher

Y die oben angegebene Bedeutung hat,

in Gegenwart eines Verdünnungsmittels und gegebenenfalls in Gegenwart einer Base umsetzt.

An die so erhaltenen Verbindungen der Formel (I) kann gegebenenfalls anschliessend eine Säure addiert werden.

Verwendet man beispielsweise 2-(2,4-Dichlorphenyl-tert.-butyl)-2-(1,2,4-triazol-1-yl-methyl)-oxiran und 1,2,4-triazol als Ausgangsstoffe, so kann der Ablauf des erfindungsgemässen Verfahrens durch das folgende Formelschema wiedergegeben werden:

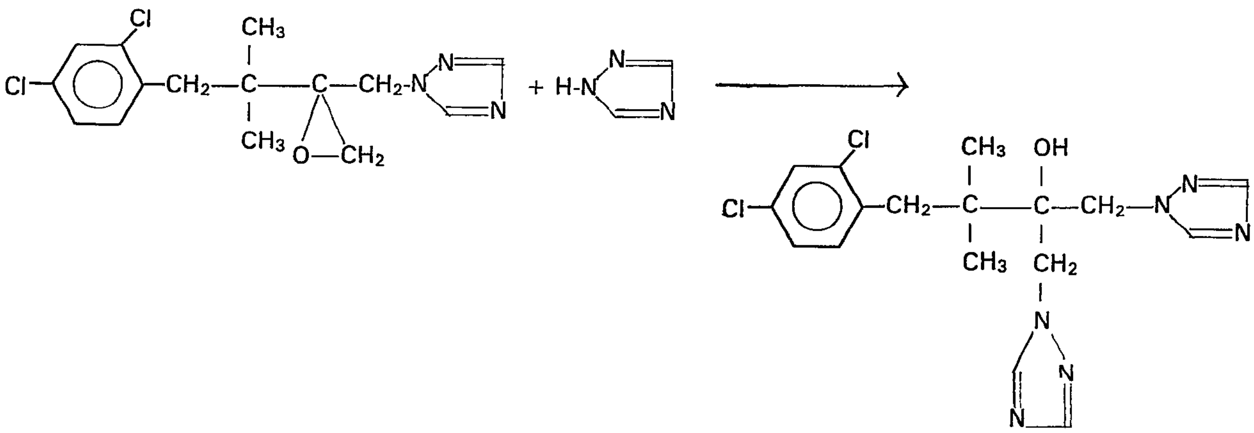

Die neuen substituierten 1,3-Diazolyl-2-propanole der Formel (I) weisen starke antimykotische Eigenschaften auf.

Ausserdem sind die neuen substituierten 1,3-Diazolyl-2-propanole interessante Zwischenprodukte.

So können z.B. die Verbindungen der allgemeinen Formel (I), in denen R für jeweils gegebenenfalls substituiertes Phenylthio, Phenylthioalkyl oder Benzylthio steht, in üblicher Weise zu den entsprechenden SO- bzw. $SO_2$-Derivaten oxidiert werden.

Die für die Durchführung des erfindungsgemässen Verfahrens als Ausgangsstoffe zu verwendenden Oxirane sind durch die Formel (II) allgemein definiert. In dieser Formel stehen, $Alk^1$, $Alk^2$, R und X vorzugsweise für die Bedeutungen, die bereits im Zusammenhang mit der Beschreibung der erfindungsgemässen Stoffe der Formel (I) vorzugsweise für diese Substituenten genannt wurden.

Die Oxirane der Formel (II) sind bekannt (vergleiche DE-OS 3 111 238); bzw. sind sie Gegenstand eigener älterer Anmeldungen, die noch nicht veröffentlicht sind (vergleiche die Deutschen Offenlegungsschriften 3 202 601 und 3 237 400; bzw. können sie in allgemein bekannter Art und Weise erhalten werden, indem man Azolyl-ketone der Formel

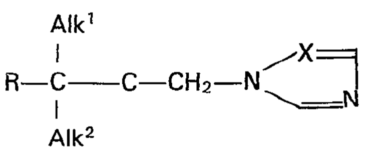 (IV)

in welcher

$Alk^1$, $Alk^2$, R und X die oben angegebene Bedeutung haben, entweder

α) mit Dimethyloxosulfonium-methylid der Formel

$$\overset{\delta+\delta-}{(CH_3)_2SOCH_2} \qquad (V)$$

in an sich bekannter Weise in Gegenwart eines Verdünnungsmittels, wie beispielsweise Dimethylsulfoxid, bei Temperaturen zwischen 20°C und 80°C umsetzt [vgl. hierzu die Angaben in J. Am. Chem. Soc. 87, 1363-1364 (1865)],

oder

β) mit Trimethylsyulfonium-methylsulfat der Formel

$$[(CH_3)_3S^+]\,CH_3SO_3^- \qquad (VI)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0°C bis 60°C, vorzugsweise bei Raumtemperatur, umsetzt [vgl. auch die Angaben in Heterocycles 8, (1977)].

Die so erhaltenen Oxirane der Formel (II) können gegebenenfalls ohne Isolierung direkt weiter umgesetzt werden.

Die Azolyl-ketone der Formel (IV) sind bekannt (vgl. DE-OS 3 048 266 und DE-OS 3 111 238), bzw. lassen sie sich nach im Prinzip bekannter Verfahren herstellen.

Die ausserdem für das erfindungsgemässe Verfahren als Ausgangsstoffe zu verwendenden Azole sind durch die Formel (III) allgemein definiert. In dieser Formel steht Y vorzugsweise für die Bedeutungen, die bereits in der Erfindungsdefinition für diesen Substituenten genannt wurden.

Als Verdünnungsmittel kommen für das erfindungsgemässe Verfahren unter den Reaktionsbedingungen inerte organische Lösungsmittel in Frage. Hierzu gehören vorzugsweise Alkoholke, wie z.B. Ethanol, Methoxyethanol oder Propanol; Ketone, wie z.B. 2-Butanon; Nitrile, wie z.B. Acetonitril.; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemässe Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid, Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemässen Verfahrens in einem grösseren Bereich variiert werden. Im allgemeinen arbeitet man bei Temperaturen zwischen 0°C und 200°C, vorzugsweise zwischen 60 und 150°C.

Bei der Durchführung des erfindungsgemässen Verfahrens setzt man vorzugsweise auf 1 Mol Oxiran der Formel (II) 1 bis 2 Mol Azol der Formel (III) und gegebenenfalls 1 bis 2 Mol Base ein; die Isolierung der Endprodukte erfolgt in allgemein üblicher Weise.

Die Verbindungen der Formel (I) können auch erhalten werden, indem man Diazolyl-ketone der Formel

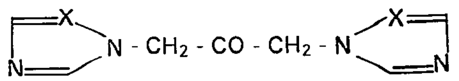

(V)

in welcher

X und Y die oben angegebene Bedeutung haben,

mit einem Grignard-Reagenz der Formel

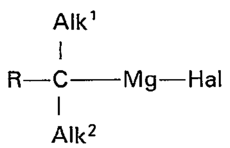

(VI)

in welcher

$Alk^1$, $Alk^2$ und R die oben angegebene Bedeutung haben und

Hal für Halogen steht,

in üblicher Weise unter den Bedingungen einer Grignard-Reaktion umsetzt,

oder indem man Dihalogenalkanole der Formel

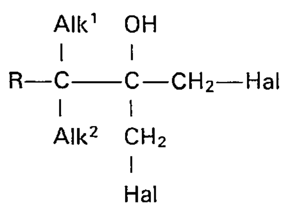

(VII)

in welcher

$Alk^1$, $Alk^2$, Hal und R die oben angegebene Bedeutung haben,

in üblicher Weise mit Azolen der Formel (III) umsetzt.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäss verwendbaren Verbindungen der Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sprosspilze sowie bi-phasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfen Keime dar, sondern hat nur erläuternden Charakter.

Als Indikationsgebiete in der Humanmedizin können beispielweise genannt werden:

Dermatomykosen und Systemmykosen durch Trichophyton mentagrophytes und andere Trichophytonarten, Mikrosporonarten, Epidermophyten floccosum, Sprosspilze und biphasische Pilze sowie Schimmelpilze hervorgerufen.

Als Indikationsgebiete in der Tiermedizin können beispielsweise aufgeführt werden:

alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form, einzelner Teile, z.B. Tabletten, Dragees. Kapseln , Pillen, Suppositorien und Ampullen vorliegen, deren Wirkstoffgehalt einem Bruchteil oder einem Vielfachen einer Einzeldosis entspricht. Die Dosierungseinheiten können z.B. 1,2,3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffe sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar-, Calciumcarbonat und Natriumcarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit

einem oder mehreren der oben angegebenen Trägerstoffe auch in mikroverkapselter Form vorliegen.

Suppositorien können neben dem oder den Wirkstoffen die üblichen wasserlöslichen oder wassunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemässen Wirkstoffe sowie von pharmazeutischen Zubreitungen, die einen oder mehrere erfindungsgemässe Wirkstoffe enthalten, in der Human- und Veterinärmedizin zur Verhütung, Besserung und/oder Heilung der oben angeführten Erkrankungen.

Die Wirkstoffe oder the pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Humanals auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

*Herstellungsbeispiele*

*Beispiel 1*

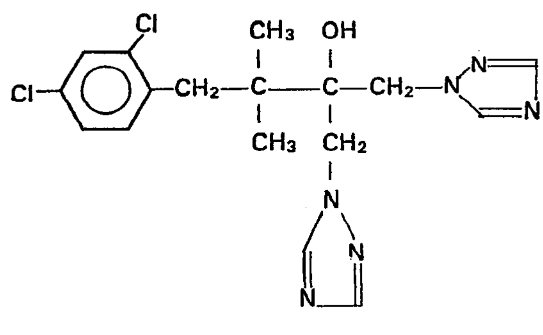

Zu einer Lösung von 0,11 g (47 mMol) Natrium in 30 ml n-Propanol werden bei Raumtemperatur unter Rühren 3,7 g (52,5 mMol) 1,2,4-Triazol gegeben. Man erhitzt auf Rückflusstemperatur und versetzt mit einer Lösung von 15,4 g (47 mMol) 2-(2,4-Dichlorphenyl-tert.-butyl)-2-(1,2,4-triazol-1-yl-methyl)-oxiran in 20 ml n-Propanol. Das Reaktionsgemisch wird 15 Stunden unter Rückfluss erhitzt, danach abgekühlt und auf Wasser gegeben. Man extrahiert mit Methylenchlorid, trocknet die organische Phase über Natriumsulfat und engt ein. Der Rückstand wird säulenchromatographisch (Kieselgel; Essigester: Cyclohexan = 3 : 1) gereinigt. Man erhält 3,5 g (18,8% der Theorie) 4-(2,4-Dichlorphenyl)-3,3-dimethyl-2-(1,2,4-triazol-1-yl-methyl)-1-(1,2,4-triazol-1-yl)-2-butanol vom Schmelzpunkt 126°C.

*Herstellung des Ausgangsproduktes*

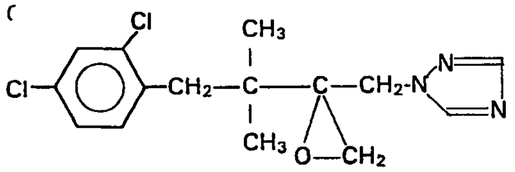

15,7 g (71,2 mMol) Trimethylsulfoxoniumiodid werden unter Stickstoffatmosphäre in 16 g Dimethylsulfoxid gelöst. Unter Kühlung wird bei Raumtemperatur mit 9,4 g (71,2 mMol) Kalium-tert.-butylat versetzt. Man lässt 6 Stunden nachrühren und versetzt anschliessend mit einer Lösung von 20 g (64,1 mMol) 4-(2,4-Dichlorphenyl)-3,3-dimethyl-1-(1,2,-4-triazol-1-yl)-2-butanon in 30 ml Tetrahydrofuran. Man lässt das Reaktionsgemisch 15 Stunden bei Raumtemperatur und 4 Stunden unter Rückfluss rühren, kühlt ab und gibt auf Wasser. Man extrahiert mit Methylenchlorid, trocknet die organische Phase über Natriumsulfat und engt im Vakuum ein. Man erhält 15,4 g (73,7% der Theorie) 2-(2,4-Dichlorphenyl-tert.-butyl)-2-(1,2,4-triazol-1-yl-methyl)-oxiran vom Brechungsindex $n_D^{20}$ = 1,5539.

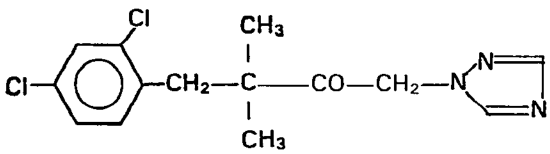

30 g (0,09 Mol) 1-Brom-4-(2,4-dichlorphenyl)-3,3-dimethyl-2-butanon, 12,4 g (0,18 Mol) 1,2,4-Triazol und 24,8 g (0,18 Mol) Kaliumcarbonat werden in 300 ml Aceton 6 Stunden unter Rückfluss erhitzt. Danach lässt man abkühlen, saugt ab und engt die Mutterlauge im Vakuum ein. Der Rückstand wird in Methylenchlorid aufgenommen, mit Wasser gewaschen, über Natriumsulfat getrocknet und im Vakuum eingeengt. Der Rückstand wird aus Diethylether umkristallisiert. Man erhält 12,8 g (45,6% der Theorie) 4-(2,4-Dichlorphenyl)-3,3-dimethyl-1-(1,2,4-triazol-1-yl)-2-butanon vom Schmelzpunkt 85°C.

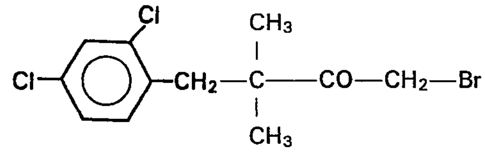

Zu einer Lösung von 64,5 g (0,26 Mol) 4-(2,4-Dichlorphenyl)-3,3-dimethyl-2-butanon in 600 ml Chloroform lässt man bei Raumtemperatur langsam 13,4 ml (0,26 Mol) Brom zutropfen. Die Reaktionslösung wird 1 Stunde bei Raumtemperatur nachgerührt. Anschliessend wird durch Abdestillieren des Lösungsmittels eingeengt. Man erhält 84,3 g (100% der Theorie) rohes 1-Brom-4-(2,4-dichlorphenyl)-3,3-dimethyl-2-butanon als Öl, das direkt weiter umgesetzt wird.

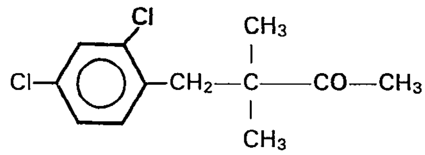

172 g (2 Mol) Methylisopropylketon, 391 g (2 Mol) 2,4-Dichlorbenzylchlorid, 20 g Tetrabutylammoniumbromid und 140 g (2,5 Mol) gepulvertes Kaliumhydroxid werden 15 Stunden unter Rückfluss erhitzt. Man lässt abkühlen und versetzt mit Wasser. Die organische Phase wird abgetrennt, über Natriumsulfat getrocknet und fraktioniert destilliert. Man erhält 129 g (26,4% der Theorie) vom Siedepunkt 90 - 95°C/0,05 mbar.

In entsprechender Weise werden die folgenden Verbindungen der allgemeinen Fprmel

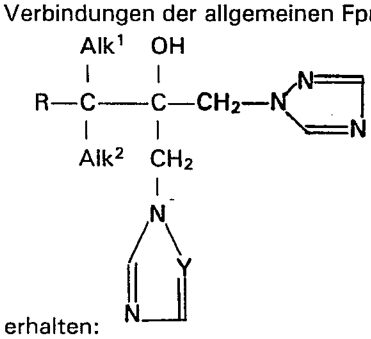

(I)

erhalten:

| Beispiel Nr. | R | Alk¹ | Alk² | X | Y | Fp (°) bzw. $n_D^{20}$ |
|---|---|---|---|---|---|---|
| 2 | 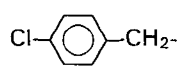 | $CH_3$ | $CH_3$ | N | N | 132 |
| 3 | 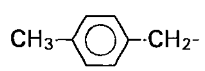 | $CH_3$ | $CH_3$ | N | N | 124 |
| 4 | 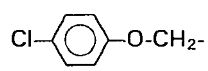 | $CH_3$ | $CH_3$ | N | N | 119-28 |
| 5 | 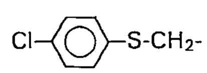 | $CH_3$ | $CH_3$ | N | N | 124 |

| Beispiel Nr. | R | $Alk^1$ | $Alk^2$ | X | Y | Fp (°) bzw. $n_D^{20}$ |
|---|---|---|---|---|---|---|
| 6 | 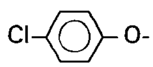 | $CH_3$ | $CH_3$ | N | N | 128 |
| 7 | 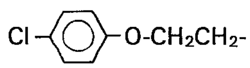 | $CH_3$ | $CH_3$ | N | N | 1,5460 |
| 8 | 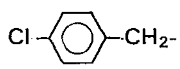 | $CH_3$ | $CH_3$ | CH | N | >220(×HCl) |
| 9 | 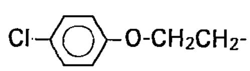 | $CH_3$ | $CH_3$ | CH | N | 1,5478 |
| 10 | 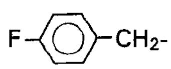 | $CH_3$ | $CH_3$ | N | N | 129 |
| 11 | 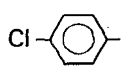 | 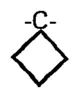 | | N | N | 78 |
| 12 | 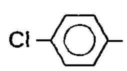 | $CH_3$ | $CH_3$ | N | N | 48 |
| 13 | 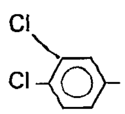 | $CH_3$ | $CH_3$ | N | N | 138 |

*Verwendungsbeispiele:*

*Beispiel A*

*Antimykotische in-vitro-Wirksamkeit*

Die in-vitro-Prüfungen wurden im Reihenverdünnungstest mit Keiminokula von durchschnittlich 5 × $10^4$ Keimen/ml Substrat durchgeführt. Als Nährmedium dienten

a) für Dermatophyten und Schimmelpilze Sabourand's milieu d'épreuve

b) für Hefen: Fleischextrakt-Traubenzucker-Bouillon.

Die Bebrütungstemperatur betrug 20°C, die Bebrütungsdauer lag bei 24 bis 96 Stunden bei Hefen und 96 Stunden bei Dermatophyten und Schimmelpilzen.
In diesem Test zeigen insbesondere die Verbindungen der Herstellungsbeispiele 1, 2, 3, 5 und 6 eine gute antimykotische Wirkung.

Tabelle A

Antimikotische in-vitro-Wirksamkeit

| | MHK-Werte in Y/ml Nährmedium bei | | | | |
|---|---|---|---|---|---|
| Wirkstoff | Trichophyton mentagr. | Microsporum canis | Candida albicans | Torulopsis glabrata | Aspergillus fumigatus |
| Verbindungen gemäss Herst. Bsp. | | | | | |
| 1 | 1 | 32 | 32 | 64 | 32 |
| 2 | 1 | 4 | 4 | 16 | 4 |
| 3 | 1 | 8 | 16 | 64 | 4 |
| 5 | 1 | 32 | 32 | 64 | 32 |
| 6 | 32 | — | 4 | 16 | 64 |

*Beispiel B*

*Antimykotische in-vivo-Wirkung (oral) bei Mäusecandiose*

*Versuchsbeschreibung:*

Mäuse vom Typ SPF-$CF_1$ wurden intravenös mit 1 - 2 × $10^6$ logarithmisch wachsenden Candida-Zellen,die in physiologischer Kochsalzlösung suspendiert werden, infiziert. Eine Stunde vor und sieben

Stunden nach der Infektion werden die Tiere mit jeweils 10 - 50 mg/kg Körpergewicht der Präparate oral behandelt.

*Ergebnis:*

Unbehandelte Tiere starben 3 bis 6 Tage post infektionem. Die Überlebensrate am 6. Tag post infektionem betrug bei unbehandelten Kontrolltieren etwa 5%.

In diesem Test zeigen insbesondere die Verbindungen der Herstellungsbeispiele 1, 2, 3, 5, 6 und 8 gute bis sehr gute Wirkung.

Zeichenerklärung:

| | | |
|---|---|---|
| + + + + + | = | sehr gute Wirkung = 90% Überlebende am 6. Tag. p.i. |
| + + + + | = | gute Wirkung = 80% Überlebende am 6. Tag p.i. |
| + + + | = | Wirkung = 60% Überlebende am 6. Tag p.i. |
| + + | = | schwache Wirkung = 40% Überlebende am 6. Tag p.i. |
| + | = | Spur Wirkung |
| k.W. | = | keine Wirkung |

Tabelle B

Antimykotische in-vivo-Wirksamkeit (oral) bei Mäusecandiose

| Wirkstoff | Wirkung |
|---|---|
| Verbindungen gemäss Herstellungs Bsp. | |
| 1 | + + + + + |
| 2 | + + + + + |
| 3 | + + + + |
| 5 | + + + + |
| 6 | + + + + |
| 8 | + + + + |

*Beispiel/Formulierungen*

1.) *Lösung*

| | |
|---|---|
| Wirkstoff gemäss Formel (I): | 10 g |
| Alkohol, rein (96%ig): | 300 g |
| Isopropylmyrisat | 526 g |
| | 836 g |

2.) *Creme:*

| | |
|---|---|
| Wirkstoff gemäss Formel (I): | 10 g |
| Arlacel 60: Sorbitan-monostearat) | 20 g |
| Tween 60: (Polyoxyethylen[20]-sorbitan monostearat) | 15 g |
| Walrat, künstlich: (Mischung von Estern von gesättigten Fettsäuren $C_{14}$-$C_{18}$ und Fettalkoholen $C_{14}$-$C_{18}$) | 30 g |
| Lanette O: (Gemisch aus Cetyl-Alkohol und Stearyl-Alkohol) | 100 g |
| Entanol G: (2-Octyl-dodecanol) | 135 g |
| Benzylalkohol: | 10 g |
| Wasser, entmineralisiert: | 680 g |
| | 1000 g |

**Patentansprüche**

1. Substituierte 1,3-Diazolyl-2-propanole der allgemeinen Formel

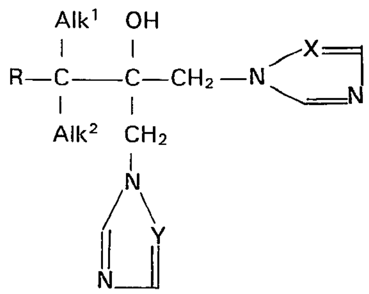

(I)

in welcher

Alk[1] für geradkettiges oder verzweigtes Alkyl mit 1 bis 4 Kohlenstoffatomen;

Alk[2] für geradkettiges oder verzweigtes Alkyl mit 1 bis 4 Kohlenstoffatomen;

Alk[1] und Alk[2] gemeinsam für einen 3- bis 7gliedrigen cycloaliphatischen Ring;

X für ein Stickstoffatom oder die CH-Gruppe;

Y für ein Stickstoffatom oder die CH-Gruppe; und

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroxyiminoalkyl mit 1 bis 4 Kohlenstoffatomen im Alkylteil, Alkoxyiminoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy,

und deren physiologisch verträgliche Säureadditionssalze.

2. Verbindungen der allgemeinen Formel (I) in Anspruch 1, in der

Alk[1] für Methyl oder Ethyl steht;

Alk² für Methyl oder Ethyl steht;

Alk¹ und Alk² gemeinsam mit dem Kohlenstoffatom an das sie gebunden sind, für Cyclobutyl, Cyclopentyl oder Cyclohexyl stehen;

X für ein Stickstoffatom oder die CH-Gruppe steht;

Y für ein Stickstoffatom oder die CH-Gruppe steht, und

R für jeweils gegenebenfalls im Phenylteil einfach oder zweifach, gleich oder verschieden substituiertes Phenyl, Benzyl, Phenethyl, Phenoxy, Phenylthio, Phenoxymethyl, Phenoxyethyl, Phenylthiomethyl, Phenylthioethyl, Benzyloxy oder Benzylthio steht, wobei als Substituenten genannt seien: Fluor, Chlor, Brom, Methyl, Isopropyl, tert.-Butyl, Methoxy, Methylthio, Trifluormethyl, Trifluormethoxy, Trifluormethylthio, Nitro, Cyano, Hydroxy, Hydroxycarbonyl, Methoxycarbonyl, Ethoxycarbonyl, Hydroxyiminomethyl, 1-Hydroxyiminoethyl, Methoximinocarbonyl, 1-Methoximinoethyl, sowie jeweils gegebenenfalls durch Fluor, Chlor, Methyl, substituiertes Phenyl, Phenoxy, Benzyl und Benzyloxy.

3. 4-(2,4-Dichlorphenyl)-3,3-dimethyl-2-(1,2,4--triazol-1-yl-methyl)-1-(1,2,4-triazol-1-yl)-2-butanol.

4. 4-(4-Chlorphenyl)-3,3-dimethyl-2-(1,2,4-triazol-1-yl-methyl)-1-(1,2,4-triazol-1-yl)-2-butanol.

5. 4-(4-Methylphenyl)-3,3-dimethyl-2)-1,2,4-triazol-1-yl-methyl)-1-(1,2,4-triazol-1-yl)-2-butanol.

6. Substituierte 1,3-Diazolyl-2-propanole der allgemeinen Formel

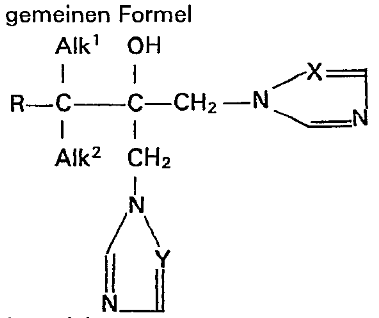

(I)

in welcher

Alk¹ für geradkettiges oder verzweigtes Alkyl mit 1 bis 4 Kohlenstoffatomen;

Alk² für geradkettiges oder verzweigtes Alkyl mit 1 bis 4 Kohlenstoffatomen;

Alk¹ und Alk² gemeinsam für einen 3- bis 7-gliedrigen cycloaliphatischen Ring;

X für ein Stickstoffatom oder die CH-Gruppe;

Y für ein Stickstoffatom oder die CH-Gruppe; und

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroxyiminoalkyl mit 1 bis 4 Kohlenstoffatomen im Alkylteil, Alkoxyiminoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy,

zur Behandlung von Krankheiten, insbesondere Mykosen.

7. Verfahren zur Herstellung von Verbindungen der allgemeinen Formel

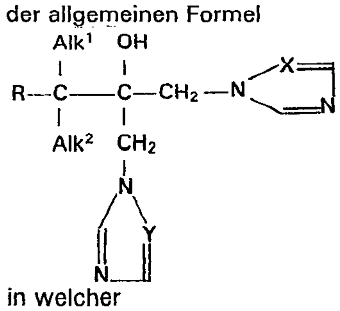

(I)

in welcher

Alk¹ für geradkettiges oder verzweigtes Alkyl mit 1 bis 4 Kohlenstoffatomen;

Alk² für geradkettiges oder verzweigtes Alkyl mit 1 bis 4 Kohlenstoffatomen;

Alk¹ und Alk² gemeinsam für einen 3- bis 7-gliedrigen cycloaliphatischen Ring;

X für ein Stickstoffatom oder die CH-Gruppe;

Y für ein Stickstoffatom oder die CH-Gruppe; und

R für jeweils gegebenenfalls im Phenylteil einfach bis dreifach, gleich oder verschieden substituiertes Phenyl, Phenylalkyl mit 1 bis 4 Kohlenstoffatomen im Alkylteil, Phenoxy, Phenylthio, Phenoxyalkyl mit 1 bis 4 Kohlenstoffatomen im Alkylteil, Phenylthioalkyl mit 1 bis 4 Kohlenstoffatomen im Alkylteil, Benzyloxy und Benzylthio steht, wobei als Substituenten vorzugsweise genannt seien: Halogen, Alkyl mit 1 bis 4 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie vorzugsweise Fluor- und Chloratomen, Nitro, Cyano, Hydroxy, Hydroxycarbonyl, Alkoxycarbonyl mit 1 bis 4 Kohlenstoffatomen im Alkylteil, Hydroxyiminoalkyl mit 1 bis 4 Kohlenstoffatomen im Alkylteil, Alkoxyiminoalkyl mit 1 bis 4 Kohlenstoffatomen in jedem Alkylteil, sowie jeweils gegebenenfalls durch Halogen und/oder Alkyl mit 1 bis 2 Kohlenstoffatomen substituiertes Phenyl, Phenoxy, Benzyl und Benzyloxy,

dadurch gekennzeichnet, dass man 2-Azolylmethyloxirane der Formel

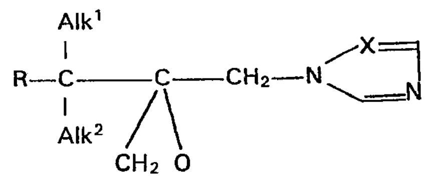

(II)

in welcher
Alk[1], Alk[2], R und X die oben angegebene Bedeutung haben,
mit Azolen der Formel

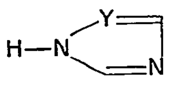

(III)

in welcher
Y die oben angegebene Bedeutung hat,
in Gegenwart eines Verdünnungsmittels und gegebenenfalls in Gegenwart einer Base umsetzt.

8. Arzneimittel, gekennzeichnet durch einen Gehalt an mindestens einer Verbindung gemäss Anspruch 1

9. Verfahren zur Herstellung von antimykotischen Mitteln, dadurch gekennzeichnet, dass man Verbindungen gemäss Anspruch 1 mit inerten, nichttoxischen, pharmazeutisch geeigneten Trägerstoffen vermischt.

**Claims**

1. Substituted 1,3-diazolyl-2-propanols of the general formula

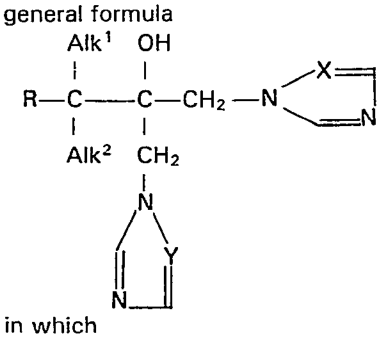

(I)

in which
$Alk_1$ represents straight-chain or branched alkyl with 1 to 4 carbon atoms;
$Alk^2$ represents straight-chain or branched alkyl with 1 to 4 carbon atoms;
$Alk^1$ and $Alk^2$ together represent a 3- to 7-membered cycloaliphatic ring;
X represents a nitrogen atom or the CH group;
Y represents a nitrogen atom or the CH group; and
R represents phenyl, phenylalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, preferred substituents which may be mentioned being; halogen, alkyl with 1 to 4 carbon atoms, alkoxy and alkylthio with in each case 1 to 4 carbon atoms, halogenoalkyl and halogenoalkoxy and halogenoalkylthio with in each case 1 to 2 carbon and 1 to 5 identical or different halogen atoms, such as preferably fluorine and chlorine atoms, nitro, cyano, hydroxyl, hydroxycarbonyl, alkoxycarbonyl with 1 to 4 carbon atoms in the alkyl part, hydroxyiminoalkyl with 1 to 4 carbon atoms in the alkyl part, alkoxyiminoalkyl with 1 to 4 carbon atoms in each alkyl part, and phenyl, phenoxy, benzyl or benzyloxy, each of which is optionally substituted by halogen and/or alkyl with 1 to 2 carbon atoms,

and physiologically tolerated acid addition salts thereof.

2. Compounds of the general formula (I) in claim 1, in which
$Alk^1$ represents methyl or ethyl
$Alk^2$ represents methyl or ethyl;
$Alk^1$ and $Alk^2$, together with the carbon atom to which they are bonded, represent cyclobutyl, cyclopentyl or cyclohexyl,
X represents a nitrogen atom or the CH group,
Y represents a nitrogen atom or the CH group, and
R represents phenyl, benzyl, phenethyl, phenoxy, phenylthio, phenoxymethyl, phenoxyethyl, phenylthiomethyl, phenylthioethyl, benzyloxy or benzylthio, each of which is optionally mono- to disubstituted in the phenyl part by identical or different substituents, substituents which may be mentioned being: fluorine, chlorine, bromine, methyl, isopropyl, tert.-butyl, methoxy, methylthio, trifluoromethyl, trifluoromethoxy, trifluoromethylthio, nitro, cyano, hydroxyl, hydroxycarbonyl, methoxycarbonyl, ethoxycarbonyl, hydroxyiminomethyl, 1-hydroxyiminoethyl, methoximinomethyl, 1-methoximinoethyl, and phenyl, phenoxy, benzyl and benzyloxy, each of which is optionally substituted by fluorine, chlorine or methyl.

3. 4-(2,4-Dichlorophenyl)-3,3-dimethyl-2-(1,2,-4-triazol-1-ylmethyl)-1-(1,2,4-triazol-1-yl)-2-butanol.

4. 4-(4-Chlorophenyl)-3,3-dimethyl-2-(1,2,4-triazol-1-yl-methyl)-1-(1,2,4-triazol-1-yl)-2-butanol.

5. 4-(4-Methylphenyl)-3,3-dimethyl-2-(1,2,4-triazol-1-yl-methyl)-1-(1,2,4-triazol-1-yl)-2-butanol.

6. Substituted 1,3-diazolyl-2-propanols of the general formula

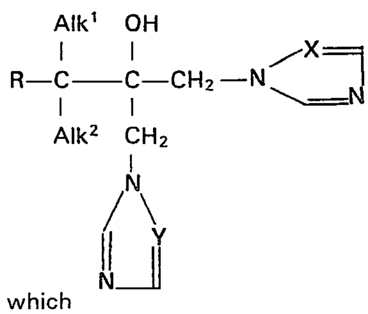

(I)

in which
$Alk^1$ represents straight-chain or branched alkyl with 1 to 4 carbon atoms;
$Alk^2$ represents straight-chain or branched alkyl with 1 to 4 carbon atoms;
$Alk^1$ and $Alk^2$ together represent a 3- to 7-membered cycloaliphatic ring;
X represents a nitrogen atom or the CH group;
Y represents a nitrogen atom or the CH group; and
R represents phenyl, phenylalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, preferred substituents which may be mentioned being; halogen, alkyl with 1 to 4 carbon atoms, alkoxy and alkylthio with in each case 1 to 4 carbon atoms, halogenoalkyl and halogenoalkoxy and halogenoalkylthio with in each case 1 to 2 carbon and 1 to 5 identical or different halogen atoms, such as preferably fluorine and chlorine atoms, nitro, cyano, hydroxyl, hydroxycarbonyl, alkoxycarbonyl with 1 to 4 carbon atoms in the alkyl part, hydroxyiminoalkyl with 1 to 4 carbon atoms in the alkyl part, alkoxyiminoalkyl with 1 to 4 carbon atoms in each alkyl part, and phenyl, phenoxy, benzyl or benzyloxy, each of which is optionally substituted by halogen and/or alkyl with 1 to 2 carbon atoms,
for treating diseases, in particular mycoses.

7. Process for the preparation of compounds of the general formula

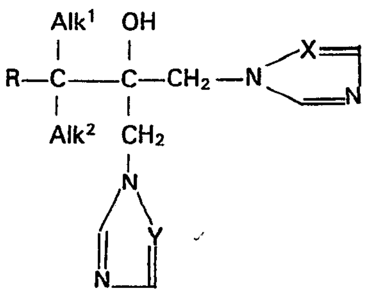
(I)

in which
$Alk^1$ represents straight-chain or branched alkyl with 1 to 4 carbon atoms;
$Alk^2$ represents straight-chain or branched alkyl with 1 to 4 carbon atoms;
$Alk^1$ and $Alk^2$ together represent a 3- to 7-membered cycloaliphatic ring;
X represents a nitrogen atom or the CH group;
Y represents a nitrogen atom or the CH group; and
R represents phenyl, phenylalkyl with 1 to 4 carbon atoms in the alkyl part, phenoxy, phenylthio, phenoxyalkyl with 1 to 4 carbon atoms in the alkyl part, phenylthioalkyl with 1 to 4 carbon atoms in the alkyl part, benzyloxy and benzylthio, each of which is optionally mono- to trisubstituted in the phenyl part by identical or different substituents, preferred substituents which may be mentioned being: halogen, alkyl with 1 to 4 carbon atoms, alkoxy and alkylthio with in each case 1 to 4 carbon atoms, halogenoalkyl and halogenoalkoxy and halogenoalkylthio with in each case 1 to 2 carbon and 1 to 5 identical or different halogen atoms, such as preferably fluorine and chlorine atoms, nitro, cyano, hydroxyl, hydroxycarbonyl, alkoxycarbonyl with 1 to 4 carbon atoms in the alkyl part, hydroxyiminoalkyl with 1 to 4 carbon atoms in the alkyl part, alkoxyiminoalkyl with 1 to 4 carbon atoms in each alkyl part, and phenyl, phenoxy, benzyl or benzyloxy, each of which is optionally substituted by halogen and/or alkyl with 1 to 2 carbon atoms,

characterised in that 2-azolylmethyl-oxirans of the formula

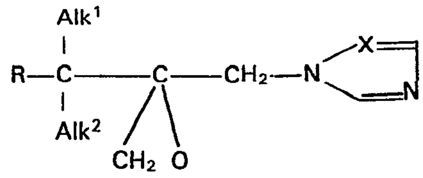
(II)

in which
$Alk^1$, $Alk^2$, R and X have the abovementioned meaning,
are reacted with azoles of the formula

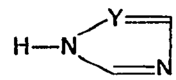
(III)

in which
Y has the abovementioned meaning,
in the presence of a diluent and if appropriate in the presence of a base.

8. Medicaments, characterised in that they contain at least one compound according to claim 1.

9. Process for the preparation of antimycotic agents, characterised in that compounds according to claim 1 are mixed with inert, non-toxic, pharmaceutically suitable excipients.

**Revendications**

1. 1,3-diazolyl-2-propanols substitués, de formule générale (I):

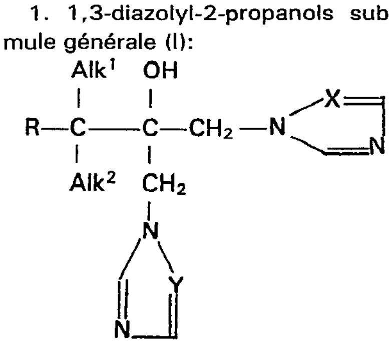
(I)

dans laquelle
$Alk^1$ représente un groupe alkyle linéaire ou ramifié ayant 1 à 4 atomes de carbone;
$Alk^2$ représente un groupe alkyle linéaire ou ramifié ayant 1 à 4 atomes de carbone;
$Alk^1$ et $Alk^2$ forment ensemble un noyau cycloaliphatique comportant 3 à 7 chaînons;
X représente un atome d'azote ou le groupe CH;
Y représente un atome d'azote ou le groupe CH; et
R représente un groupe phényle, phénylalkyle ayant 1 à 4 atomes de carbone dans la partie alkyle, phénoxy, phénylthio, phénylalkyle ayant 1 à 4 atomes de carbone dans la partie alkyle, phénylthioalkyle ayant 1 à 4 atomes de carbone dans la partie alkyle, benzyloxy et benzylthio, chacun éventuellement substitué une à trois fois, de façon identique ou différente, dans la partie phényle, et l'on peut citer de préférence comme substituants: un halogène, un groupe alkyle ayant 1 à 4 atomes de carbone, alkoxy et alkylthio ayant chacun 1 à 4 atomes de carbone, halogénoalkyle et halogénoalcoxy ainsi que halogénoalkylthio ayant chacun 1 à 2 atomes de

carbone et 1 à 5 atomes d'halogène identiques ou différents, comme de préférence des atomes de fluor et de chlore, un groupe nitro, cyano, hydroxy, hydroxycarbonyle, alcoxycarbonyle ayant 1 à 4 atomes de carbone dans la partie alkyle, hydroxyiminoalkyle ayant 1 à 4 atomes de carbone dans la partie alkyle, alcoxyiminoalkyle ayant 1 à 4 atomes de carbone dans chaque partie alkyle, ainsi qu'un groupe phényle, phénoxy, benzyle ou benzyloxy, substitué à chaque fois par de l'halogène et/ou par un groupe alkyle ayant 1 à 2 atomes de carbone;

et leurs sels d'addition d'acides physiologiquement tolérables.

2. Composés de formule générale (I) de la revendication 1, dans laquelle:

$Alk^1$ représente un groupe méthyle ou éthyle;

$Alk^2$ représente un groupe méthyle ou éthyle;

$Alk^1$ et $Alk^2$ forment, pris ensemble avec l'atome de carbone auquel ils sont reliés, un groupe cyclobutyle, cyclopentyle ou cyclohexyle;

X représente un atome d'azote ou le groupe CH;

Y représente un atome d'azote ou le groupe CH; et

R représente un groupe phényle, benzyle, phénéthyle, phénoxy, phénylthio, phénoxyméthyle, phénoxyéthyle, phénylthiométhyle, phénylthioéthyle, benzyloxy ou benzylthio, éventuellement substitués chacun dans la partie phényle, une à deux fois, de façon identique ou différente, et l'on peut citer comme substituants: un atome de fluor, de chlore, de brome, un groupe méthyle, isopropyle, tertiobutyle, méthoxy, méthylthio, trifluorométhyle, trifluorométhoxy, trifluorométhylthio, nitro, cyano, hydroxyle, hydroxycarbonyle, méthoxycarbonyle, éthoxycarbonyle, hydroxyiminométhyle, 1-hydroxyiminoéthyle, méthoxyiminométhyle, 1-méthoxyiminoéthyle, ainsi qu'un groupe phényle, phénoxy, benzyle et benzyloxy, éventuellement substitués chacun par un atome de fluor ou de chlore ou un groupe méthyle.

3. Le 4-(2,4-dichlorophényl)-3,3-diméthyl-2-(1,2,4-triazol-1-yl-méthyl)-1-(1,2,4-triazol-1-yl)-2-butanol.

4. Le 4-(4-chlorophényl)-3,3-diméthyl-2-(1,2,4-triazol-1-yl-méthyl)-1-(1,2,4-triazol-1-yl)-2-butanol.

5. Le 4-(4-méthylphényl)-3,3-diméthyl-2-(1,2,4-triazol-1-yl-méthyl)-1-(1,2,4-triazol-1-yl)-2-butanol.

6. 1,3-diazolyl-2-propanols substitués de formule générale:

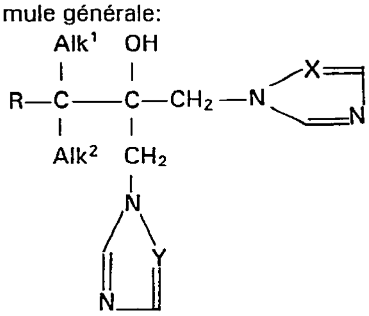

(I)

dans laquelle

$Alk^1$ représente un groupe alkyle linéaire ou ramifié ayant 1 à 4 atomes de carbone;

$Alk^2$ représente un groupe alkyle linéaire ou ramifié ayant 1 à 4 atomes de carbone;

$Alk^1$ et $Alk^2$ forment ensemble un noyau cycloaliphatique comportant 3 à 7 chaînons;

X représente un atome d'azote ou le groupe CH;

Y représente un atome d'azote ou le groupe CH; et

R représente un groupe phényle, phénylalkyle ayant 1 à 4 atomes de carbone dans la partie alkyle, phénoxy, phénylthio, phénylalkyle ayant 1 à 4 atomes de carbone dans la partie alkyle, phénylthioalkyle ayant 1 à 4 atomes de carbone dans la partie alkyle, benzyloxy et benzylthio, éventuellement substitué chacun dans la partie phényle une à trois fois, de façon identique ou différente, et l'on peut citer comme substituants de préférence: un atome d'halogène, un groupe alkyle ayant 1 à 4 atomes de carbone, alcoxy et alkylthio ayant chacun 1 à 4 atomes de carbone, halogénoalkyle et halogénoalcoxy ainsi que halogénoalkylthio ayant chacun 1 à 2 atomes de carbone et 1 à 5 atomes d'halogène identiques ou différents, comme de préférence des atomes de fluor et de chlore, un groupe nitro, cyano, hydroxy, hydroxycarbonyle, alc-oxycarbonyle ayant 1 à 4 atomes de carbone dans la partie alkyle, hydroxyiminoalkyle ayant 1 à 4 atomes de carbone dans la partie alkyle, alcoxyiminoalkyle ayant 1 à 4 atomes de carbone dans chaque partie alkyle, ainsi qu'un groupe phényle, phénoxy, benzyle ou benzyloxy, chacun éventuellement substitué par de l'halogène et/ou par un groupe alkyle ayant 1 à 2 atomes de carbone;

pour traiter des maladies, en particulier des mycoses.

7. Procédé pour préparer les composés de formule générale:

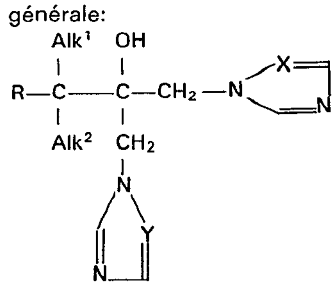

(I)

dans laquelle

$Alk^1$ représente un groupe alkyle linéaire ou ramifié ayant 1 à 4 atomes de carbone;

$Alk^2$ représente un groupe alkyle linéaire ou ramifié ayant 1 à 4 atomes de carbone;

$Alk^1$ et $Alk^2$ forment ensemble un noyau cycloaliphatique comportant 3 à 7 chaînons;

X représente un atome d'azote ou le groupe CH;

Y représente un atome d'azote ou le groupe CH; et

R représente un groupe phényle, phénylalkyle ayant 1 à 4 atomes de carbone dans la partie alkyle, phénoxy, phénylthio, phénylalkyle ayant 1 à 4 atomes de carbone dans la partie alkyle, phénylthioalkyle ayant 1 à 4 atomes de carbone dans la partie alkyle, benzyloxy et benzylthio, chacun éventuellement substitué une à trois fois, de façon identique ou différente, dans la partie phényle, et l'on peut citer de préférence comme substituants: un halogène, un groupe alkyle ayant 1 à 4 atomes de carbone, alkoxy et alkylthio ayant chacun 1 à 4 atomes de carbone, halogénoalkyle et halogénoalcoxy ainsi que halogénoalkylthio ayant chacun 1 à 2 atomes de

carbone et 1 à 5 atomes d'halogène identiques ou différents, comme de préférence des atomes de fluor et de chlore, un groupe nitro, cyano, hydroxy, hydroxycarbonyle, alcoxycarbonyle ayant 1 à 4 atomes de carbone dans la partie alkyle, hydroxyiminoalkyle ayant 1 à 4 atomes de carbone dans la partie alkyle, alcoxyiminoalkyle ayant 1 à 4 atomes de carbone dans chaque partie alkyle, ainsi qu'un groupe phényle, phénoxy, benzyle ou benzyloxy, chacun éventuellement substitué par de l'halogène et/ou par un groupe alkyle ayant 1 à 2 atomes de carbone;

caractérisé en ce qu'on fait réagir, en présence d'un diluant et éventuellement en présence d'une base, des 2-azolylméthyloxirannes de formule:

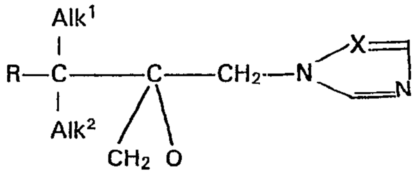

(II)

dans laquelle:

$Alk^1$, $Alk^2$, R et Y ont le sens précité,

avec des azoles de formule:

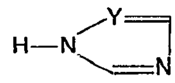

(III)

dans laquelle:

Y a les sens précité.

8. Médicament, caractérisé par une teneur en au moins un composé selon la revendication 1.

9. Procédé pour préparer des produits antimycosiques, caractérisé en de qu'on mélange des composés selon la revendication 1, avec des excipients ou véhicules inertes, pharmaceutiquement convenables, non toxiques.